Europäisches Patentamt

European Patent Office

Office européen des brevets

(11) Numéro de publication: **0 418 147 A1**

## DEMANDE DE BREVET EUROPEEN

(21) Numéro de dépôt: 90402501.2

(22) Date de dépôt: 11.09.90

(51) Int. Cl.⁵: **A61B 1/12, A61M 25/10, A61B 17/22**

(30) Priorité: 13.09.89 FR 8911966

(43) Date de publication de la demande:
20.03.91 Bulletin 91/12

(84) Etats contractants désignés:
CH DE GB IT LI

(71) Demandeur: **Croisy, Renaud**
**15 rue Marignac**
**CH-1206 Geneve(CH)**

Demandeur: **Croisy, Nicolas**
**15 rue Marignac**
**CH-1206 Geneve(CH)**

(72) Inventeur: **Croisy, Renaud**
**15 rue Marignac**
**CH-1206 Geneve(CH)**
Inventeur: **Croisy, Nicolas**
**15 rue Marignac**
**CH-1206 Geneve(CH)**

(74) Mandataire: **Mongrédien, André et al**
**c/o SOCIETE DE PROTECTION DES**
**INVENTIONS 25, rue de Ponthieu**
**F-75008 Paris(FR)**

(54) Catheter d'observation et/ou d'intervention dans un conduit du corps humain parcouru par un liquide opaque.

(57) Cathéter d'observation et/ou d'intervention dans un conduit du corps humain parcouru par un liquide opaque, comprenant une gaine (8) de forme générale cylindrique allongée munie de canaux de passage (12, 14, 16, 18) pour des moyens d'éclairage, des moyens de vision, des moyens d'amenée et d'extraction d'un liquide d'irrigation et/ou des moyens d'intervention, caractérisé en ce que la gaine (8) est montée coulissante dans une enveloppe cylindrique externe (20) portant, au voisinage de son extrémité et sur sa paroi externe, un premier ballonnet annulaire gonflable (22) permettant, lorsqu'il est sous pression et vient prendre appui sur la paroi du conduit (2) du corps, de fixer cette enveloppe externe (20) en translation par rapport au conduit (2) et d'interdire la circulation du liquide opaque dans la région d'observation et/ou d'intervention.

FIG. 1

## CATHETER D'OBSERVATION ET/OU D'INTERVENTION DANS UN CONDUIT DU CORPS HUMAIN PARCOU-RU PAR UN LIQUIDE OPAQUE

La présente invention se rapporte d'une façon générale au domaine médical de l'observation et du traitement du corps humain à l'aide de sondes endoscopiques que l'on introduit à travers un conduit interne naturel ou artificiel.

On connait déjà des sondes ou endoscopes permettant d'effectuer des observations et/ou certains traitements dans des zones déterminées d'une cavité du corps humain. De telles sondes sont utilisées couramment, par exemple en urologie et en gastro-entérologie. Dans leur constitution actuelle la plus générale, elles comportent principalement dans une gaine cylindrique en matière synthétique ou plastique servant de cathéter et s'étendant entre une tête d'observation et de commande externe manoeuvrée par le chirurgien et une extrémité d'intervention interne, un certain nombre de conduits creux ou canaux longitudinaux, destinés à recevoir des moyens d'éclairage, des moyens d'observation et des moyens de traitement.

La présente invention s'applique tout particulièrement à un cathéter de cette nature destiné à l'observation et/ou à l'intervention dans un conduit du corps humain parcouru par un liquide opaque tel que le sont, à des degrés divers par exemple, le sang des vaisseaux, l'urine du système urinaire ou la bile. Elle a surtout pour but d'apporter une contribution importante au problème de la vision correcte dans de tels milieux opaques.

De façon connue, lorsque l'on désire travailler dans un tel milieu, on spécialise l'un des canaux de passage du cathéter dans l'apport et l'extraction d'un liquide physiologique transparent d'irrigation qui a pour objet de créer, aux instants où il est délivré dans le milieu en cours d'examen, des flashes de clarté qui rendent l'observation possible et l'intervention, par exemple avec une fibre laser, beaucoup plus sûre.

A l'aide des cathéters connus jusqu'à ce jour toutefois, l'éclaircissement obtenu dans le milieu en cours d'observation est malheureusement très fugitif, car le sang, l'urine ou la bile circulant en permanence dans le conduit en cours d'examen ont tendance à reconstituer rapidement l'opacité initiale. Cette façon de procéder nécessite par conséquent l'injection de flashes répétés et successifs de liquide d'irrigation qui peuvent conduire à l'introduction dans ce même conduit du corps humain, de quantités de liquide d'irrigation non négligeables pouvant atteindre par exemple 1,8 l à 2 l au cours d'une même intervention.

Ceci peut être parfois préjudiciable pour le patient sans permettre par ailleurs d'avoir une bonne vision prolongée lors de l'observation ou de l'intervention dans le conduit examiné.

D'autres techniques beaucoup plus complexes et reposant sur le traitement d'images par des moyens informatiques ont été envisagées pour permettre une bonne vision en milieu opaque, sans toutefois conduire à des résultats pratiquement utilisables jusqu'à ce jour.

La présente invention a précisément pour objet un cathéter d'observation et/ou d'intervention en milieu opaque qui permet de s'affranchir des difficultés précédemment rappelées à l'aide de moyens dont la mise en oeuvre est particulièrement simple.

Ce cathéter d'observation et/ou d'intervention dans un conduit du corps humain parcouru par un liquide opaque, comprenant une gaine de forme générale cylindrique allongée munie de canaux de passage pour des moyens d'éclairage, des moyens de vision, des moyens d'amenée et d'extraction d'un liquide d'irrigation et/ou des moyens d'intervention, se caractérise en ce que la gaine est montée coulissante dans une enveloppe cylindrique externe portant, au voisinage de son extrémité et sur sa paroi externe, un premier ballonnet annulaire gonflable permettant, lorsqu'il est sous pression et vient prendre appui sur la paroi du conduit du corps, de fixer cette enveloppe externe en translation par rapport au conduit et d'interdire la circulation du liquide opaque dans la région d'observation et/ou d'intervention.

La présence d'un ballonnet annulaire gonflable situé au voisinage de l'extrémité sur la paroi externe de la gaine du cathéter permet, par conséquent, d'interdire le moment venu la circulation du liquide opaque dans la région d'observation, en bloquant cette circulation entre la gaine et les parois du conduit du corps humain en cours d'examen. Lorsque la circulation du liquide opaque est ainsi bloquée, les injections de liquide d'irrigation effectuées par l'un des canaux du cathéter produisent des dilutions et par là-même des zones d'éclaircie dans ce même liquide opaque dont l'importance et la durée sont sans commune mesure avec celles des flashes que l'on obtenait dans la technique antérieure. C'est là un énorme progrès dans l'utilisation des techniques d'observation endoscopiques en milieu liquide opaque et le ballonnet annulaire gonflable a, de surcroît , l'avantage, en prenant appui sur la paroi du conduit du corps en examen, de fixer l'enveloppe externe de la gaine en translation par rapport au conduit. Ceci autorise, par conséquent, le coulissement de la gaine à l'intérieur de cette enveloppe en cours d'intervention d'une longueur de 10 à 15 cm par exemple, permettant ainsi une translation de cette gaine vers l'obstacle à détruire (athérome, rétrécissement) et une progression ultérieure de celle-ci au-delà de chaque obstacle lorsqu'il a été supprimé par les moyens d'intervention.

Le ballonnet annulaire gonflable pouvant être à volonté mis sous pression ou vidé d'air depuis la tête d'observation et de commande externe de l'endoscope, il est donc possible, en le fixant successivement sur la paroi du conduit par déplacements successifs de 10 à 15 cm, de propulser la gaine, dans des conditions de bonne visibilité et d'intervention fiable, sur une grande distance tout le long du conduit du corps humain à traiter.

Les dimensions de l'enveloppe cylindrique externe par rapport à la gaine ne sont pas critiques et il suffit simplement que cette enveloppe autorise le coulissement facile de la gaine à l'intérieur de l'enveloppe préalablement fixée sur la paroi du conduit à l'aide du ballonnet.

Selon l'invention toutefois, on a trouvé avantageux de constituer cette enveloppe cylindrique externe en une matière plastique et de lui donner un diamètre qui soit supérieur de 0,1 à 0,2 mm à celui de la gaine elle-même.

Selon une autre caractéristique également très importante de la présente invention, l'espace intermédiaire entre la gaine et son enveloppe cylindrique externe est également munie d'un deuxième ballonnet gonflable annulaire permettant, lorsqu'il est sous pression, de fixer l'état de translation de la gaine dans son enveloppe et d'interdire toute fuite de liquide opaque dans l'espace intermédiaire entre la gaine et son enveloppe.

Ce deuxième ballonnet gonflable annulaire de très faibles dimensions mais néanmoins relié comme le premier ballonnet par des moyens de gonflage à l'extérieur du système d'observation, remplit une double fonction technique. D'une part, il bloque en translation la gaine dans son enveloppe lorsqu'il est dans la position sous pression et d'autre part il améliore encore l'étanchéité du système en empêchant toute fuite de liquide opaque qui pourrait sans précaution spéciale se produire encore dans l'espace intermédiaire entre la gaine et son enveloppe. Lorsqu'ainsi les deux ballonnets annulaires sont simultanément sous pression, toute circulation du liquide opaque est interdite dans la partie du conduit du corps humain où se trouve le cathéter d'observation.

Le deuxième ballonnet gonflable annulaire situé dans l'espace intermédiaire entre la gaine et son enveloppe cylindrique externe peut être indifféremment fixé soit sur la paroi externe de la gaine, soit sur la paroi interne de l'enveloppe cylindrique externe. On conçoit en effet que ces deux options puissent être envisagées sans pour autant compromettre l'obtention de la double fonction technique que l'on attend du deuxième ballonnet gonflable.

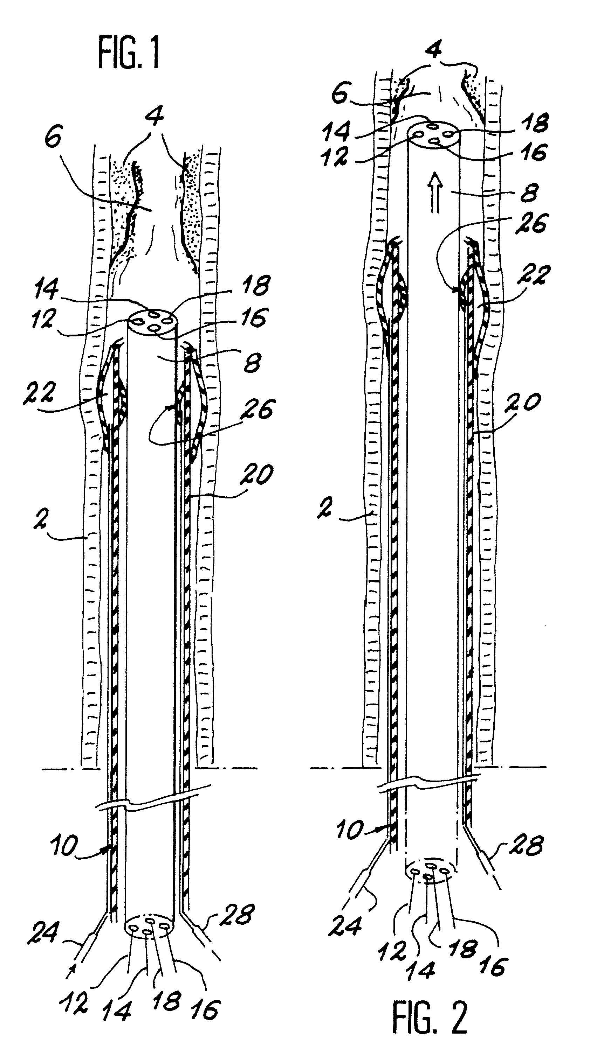

De toutes façons, l'invention sera mieux comprise en se référant à la description qui suit d'un exemple de mise en oeuvre du cathéter objet de l'invention, description qui sera faite à titre illustratif et non limitatif en se référant aux figures 1 et 2 ci-jointes sur lesquelles :

- la figure 1 montre la progression d'un cathéter conforme à l'invention dans un vaisseau sanguin, le cathéter étant pratiquement recouvert de sa gaine extérieure ;

- la figure 2 montre le même cathéter dans un état de progression plus avancé, après qu'il soit sorti d'une certaine quantité de l'extrémité de sa gaine externe.

La figure 1 représente en coupe schématique le cathéter objet de l'invention après son introduction dans un vaisseau sanguin 2 dont les parois comportent des plaques d'athéromes 4 que l'on localise d'abord pour les détruire ensuite à l'aide d'une fibre véhiculant un faisceau laser. L'intervention étant faite in vivo, du sang circule librement à l'intérieur 6 du vaisseau 2.

De façon connue, le cathéter objet de l'invention se compose essentiellement d'une gaine 8 en matière plastique de forme générale cylindrique s'étendant depuis l'extrémité d'intervention jusqu'à la tête d'observation et de commande externe 10. De façon également connue, cette gaine 8 comporte un certain nombre de canaux de passage, au nombre de quatre dans l'exemple décrit, et référencés respectivement 12, 14, 16 et 18, chacun de ces canaux est affecté au passage de l'un des moyens choisis parmi les moyens d'éclairage, les moyens de vision, les moyens d'amenée et d'extraction d'un liquide d'irrigation et les moyens d'intervention, par exemple une fibre laser.

Conformément à l'invention, la gaine 8 est montée coulissante dans une enveloppe externe 20 également en matière plastique, laquelle est munie au voisinage de son extrémité et sur sa paroi externe d'un premier ballonnet annulaire 22 gonflable à partir d'une introduction d'air 24 située au niveau de la tête de commande 10. Sur le dessin, les proportions diamétrales de la gaine 8 et de l'enveloppe externe 20 n'ont pas été respectées, et ce de façon intentionnelle pour permettre une meilleure lecture. Dans la réalité, on peut donner à titre d'exemple non limitatif les dimensions suivantes pour les différents organes en présence : si le vaisseau sanguin 2 a par exemple un diamètre de 1,5 cm, la gaine 8 peut avoir un diamètre de 1 cm et l'espace annulaire entre cette gaine 8 et l'enveloppe externe 20 une dimension radiale de 0,1 à 0,2 mm d'épaisseur. L'espace laissé libre entre la gaine 8 et la paroi du vaisseau 2 pour le ballonnet 22 lorsqu'il est gonflé, peut être par exemple de l'ordre de 2,5 mm.

Dans le cas de l'exemple de mise en oeuvre décrit sur les figures 1 et 2, le cathéter objet de l'invention comporte également un deuxième ballonnet gonflable annulaire 26 situé dans l'espace intermédiaire entre la gaine 8 et son enveloppe cylindrique externe 20. Ce deuxième ballonnet gonflable 26 est également relié à des moyens 28 d'introduction d'air sous pression au niveau de la tête 10 d'observation du cathéter. Dans l'exemple décrit, ce deuxième ballonnet gonflable 26 est fixé sur la paroi interne de l'enveloppe cylindrique externe 20, mais il doit bien être entendu qu'il est aussi possible de le fixer le cas échéant directement sur la paroi externe de la gaine 8.

Le dispositif décrit sur les figures 1 et 2 fonctionne de la façon suivante :

Lors de l'approche d'une cible de plaques d'athéromes 4 à détruire (Fig. 1), la gaine 8 est introduite dans le vaisseau sanguin 2 munie de son enveloppe cylindrique externe 20 la recouvrant pratiquement jusqu'à l'extrémité.

A ce moment, l'opérateur qui commande le système depuis la tête d'intervention 10, déclenche la mise sous pression et le gonflage du ballonnet 22, ce qui provoque ainsi l'obturation du vaisseau 2 et interrompt la majeure partie de la circulation sanguine dans l'espace 6. Pour parfaire cette obturation, l'opérateur gonfle également depuis l'extérieur par l'ouverture 28 le deuxième ballonnet gonflable 26, ce qui a pour effet d'obturer complètement l'espace intermédiaire entre la gaine 8 et son enveloppe externe 20 interrompant ainsi toute circulation du sang dans le vaisseau 2 entre l'amont et l'aval de la tête d'intervention.

Dans l'état précédent où les deux ballonnets 22 et 26 sont simultanément gonflés, non seulement l'arrêt de la circulation sanguine dans l'espace 6 est interrompu, mais l'état de translation de l'enveloppe 20 dans ce même vaisseau 2 et de la gaine 8 dans l'enveloppe externe 20 est fixé, permettant ainsi une vision et une intervention dans de bonnes conditions à l'intérieur du vaisseau 2. En effet, si, par le canal d'irrigation, on envoie à ce moment dans l'espace 6 un certain volume de liquide clair, la qualité de la vision dans le liquide opaque qu'est le sang, subsistera pendant un moment relativement important, de plusieurs dizaines de secondes par exemple permettant ainsi une vision très confortable et un tir très précis de la fibre laser sur les athéromes 4.

Lorsque cette étape est franchie, et si l'on veut progresser plus loin pour continuer le travail dans le vaisseau 2, on procède de la façon suivante. On dégonfle le deuxième ballonnet gonflable 26, ce qui libère la gaine 8 en translation dans son enveloppe externe 20 et lui permet de progresser vers l'avant comme indiqué sur la figure 2 d'une longueur d'une dizaine de centimètres environ. Ceci est particulièrement intéressant pour la raison suivante. Les tirs effectués par la fibre laser n'ont pas pour ambition de nettoyer complètement les parois du vaisseau 2 au niveau d'un athérome, mais simplement d'ouvrir dans cet athérome un passage de diamètre suffisant pour la circulation du sang. Or, ce diamètre qui, en général, ne permettrait pas la progression de l'ensemble du cathéter avec son ballonnet 22, autorise au contraire parfaitement le passage de la seule gaine 8.

Lorsque l'on désire enfin progresser davantage, on dégonfle également le ballonnet 22 et l'on transporte tout l'ensemble de la tête jusqu'à une étape ultérieure où l'on fixe à nouveau l'état de translation par un gonflage consécutif des ballonnets 22 et 26.

On voit donc que le cathéter selon l'invention équipé de ses moyens d'obturation du vaisseau sanguin visité permet, de façon très simple, d'intervenir dans des conditions de clarté inconnues jusqu'à ce jour dans le milieu opaque constitué par le sang d'un vaisseau, tout en assurant à chaque pas de la progression, une fixation et une obturation de l'enveloppe externe 20 dans le vaisseau et de la gaine 8 dans son enveloppe externe.

L'exemple décrit précédemment a été donné en se référant au cas d'un vaisseau sanguin, mais il est bien évident que tous les vaisseaux du corps humain parcourus par des liquides opaques sont susceptibles d'être traités par le cathéter objet de l'invention, tels que par exemple les voies urinaires ou le canal biliaire.

Il est facile également de constater le progrès technique réalisé par ce cathéter si l'on se remémore le fait que dans les techniques d'endoscopie antérieures, il fallait injecter sous formes de flashes répétitifs ou à l'aide de pompages permanents, des quantités de liquides transparents physiologiques énormes pouvant aller au cours d'une même intervention jusqu'à 2 litres, pour n'obtenir malgré tout qu'une qualité de vision très approximative qui ne permettait bien souvent pas d'effectuer des tirs laser au moment même où la perception lumineuse était la meilleure.

## Revendications

1. Cathéter d'observation et/ou d'intervention dans un conduit du corps humain parcouru par un liquide opaque, comprenant une gaine (8) de forme générale cylindrique allongée munie de canaux de passage (12, 14, 16, 18) pour des moyens d'éclairage, des moyens de vision, des moyens d'amenée et d'extraction d'un liquide d'irrigation et/ou des moyens d'intervention, caractérisé en ce que la gaine (8) est montée coulissante dans une enveloppe cylindrique externe (20) portant, au voisinage de son extrémité et sur sa paroi externe, un premier ballonnet annulaire gonflable (22) permettant, lorsqu'il est sous pression et vient prendre appui sur la paroi du conduit (2) du corps, de fixer cette enveloppe externe (20) en translation par rapport au conduit (2) et d'interdire la circulation du liquide opaque dans la région d'observation et/ou d'intervention et en ce que l'espace intermédiaire entre la gaine (8) et son enveloppe cylindrique externe (20) est également muni d'un deuxième ballonnet gonflable annulaire (26) permettant, lorsqu'il est sous pression, de fixer l'état de translation de la gaine (8) dans son enveloppe (20) et d'interdire toute fuite de liquide opaque dans l'espace intermédiaire entre la gaine et son enveloppe.

2. Cathéter selon la revendication 1, caractérisé en ce que l'enveloppe cylindrique externe (20) est en matière plastique et le diamètre supérieur de 0,1 à 0,2 mm à celui de la gaine.

3. Cathéter selon la revendication 1, caractérisé en ce que le deuxième ballonnet gonflable annulaire (26) est fixé sur la paroi externe de la gaine (8).

4. Cathéter selon la revendication 1, caractérisé en ce que le deuxième ballonnet gonflable annulaire (26) est fixé sur la paroi interne de l'enveloppe cylindrique externe (20).

5. Cathéter selon la revendication 1 , caractérisé en ce que chacun des premier (22) et deuxième (26) ballonnets est relié à la tête de commande (10) du cathéter par des moyens d'introduction et de transfert de l'air d'alimentation (24, 28).

# FIG. 1

# FIG. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,A | US-A-4 445 892   (H.M.G.HUSSEIN ET AL.) <br> * colonne 1, ligne 51 - colonne 2, ligne 27; figures 1-7 * <br> – – – | 1,2,3,5 | A 61 B 1/12 <br> A 61 M 25/10 <br> A 61 B 17/22 |
| P,Y | EP-A-0 371 486   (ADVANCED CARDIOVASCULAR SYS-TEMS INC.) <br> * le document en entier * <br> – – – | 1 | |
| A | WO-A-8 907 413   (TERUMO KABUSHIKI KAISHA) <br> * abrégé; figures 1-6 * <br> – – – | 1,3,5 | |
| A | US-A-4 418 688   (M.P.LOEB) <br> * abrégé; figures 1-3 * <br> – – – | 1,5 | |
| A | WO-A-8 900 407   (T.V.TAYLOR) <br> * abrégé; figures 1, 2 * <br> – – – – – | 1 | |

| DOMAINES TECHNIQUES RECHERCHES (Int. Cl.5) |
|---|
| A 61 B <br> A 61 M |

Le présent rapport de recherche a été établi pour toutes les revendications

| Lieu de la recherche | Date d'achèvement de la recherche | Examinateur |
|---|---|---|
| La Haye | 19 décembre 90 | HUNT,B.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

&: membre de la même famille, document correspondant